# EUROPEAN PATENT APPLICATION

(11) **EP 4 249 607 A1**
(43) Date of publication of application: **27.09.2023**
(21) Application number: 22163365.4
(22) Date of filing: 21.03.2022
(51) Int. Cl.: C12Q 1/6883

(54) **PANEL OF MARKERS FOR PREDICTION OF EPILEPSY RECURRENCE IN PATIENTS WITH TUBEROUS SCLEROSIS AND THE USES THEREOF**

(71) Applicant: Warszawski Uniwersytet Medyczny, 02-091 Warszawa (PL); Instytut Biologii Doswiadczalnej PAN. im. M. Nenckiego, 02-093 Warszawa (PL); Miedzynarodowy Instytut Biologii Molekularnej I Komorkowej, 02-109 Warszawa (PL); Instytut Pomnik-Centrum Zdrowia Dziecka, 04-730 Warsaw (PL)
(72) Inventor: Roura Canalda, Adria-Jaume, 02-389 Warsaw (PL); Gielniewski, Bartlomiej, 04-382 Warsaw (PL); Jaworski, Jacek, 04-421 Warsaw (PL); Jozwiak, Sergiusz, 04-783 Warsaw (PL); Kaminska-Kaczmarek, Bozena, 05-806 Komorow (PL); Kotulska-Jozwiak, Katarzyna, 04-783 Warsaw (PL); Liszewska, Ewa, 18-400 Lomza (PL); Wojtas, Bartosz, 02-796 Warsaw (PL)
(74) Representative: Kondrat, Mariusz

(57) **Abstract**

The subject of invention is a panel of RNA markers for the prediction of the occurrence of epilepsy recurrence following drug withdrawal in patients with tuberous sclerosis, characterised in that it comprises following markers P2RY13, IL13RA1, PDE4B. Another subject of the invention is an in vitro method to predict the occurrence of epilepsy recurrence following drug withdrawal in patients with tuberous sclerosis, comprising the steps of: a) Measurement of the expression level of a panel of predictive markers prior to drug withdrawal; b) Determination of the risk of epilepsy recurrence based on the level of the of markers from prediction panel, characterised in that said panel of predictive markers is a panel of RNA markers comprising P2RY13, IL13RA1, PDE4B, and the determination of the risk of epilepsy recurrence after drug withdrawal is based on altered levels of at least two predictive markers of the said panel compared to a reference level. Another subject of the invention is a use of a panel of markers according to the invention, for predicting the epilepsy recurrence following drug withdrawal in patients with tuberous sclerosis according to the method according to the invention.

## Description

The subject of the invention is a panel of markers for the prediction of the epilepsy recurrence in patients with tuberous sclerosis, an in vitro method for the prediction of the epilepsy recurrence in patients with tuberous sclerosis, and the use of a panel of markers in this method.

Tuberous sclerosis (TS), also known as Tuberous Sclerosis Complex (TSC) or Bourneville-Pringle disease, belongs to the so-called neurocutanous diseases. TSC is caused by a mutation in the TSC1 or TSC2 genes, which encode essential components of a protein complex that controls the activity of the cell metabolism regulator - mammalian target of rapamycin (mTOR). About one person in 6,000 is born with such mutation (the current number of patients worldwide is estimated to be about 1 million people). However, the clinical picture of TSC can vary greatly and it can present differently even in people with the same mutation. The most common manifestations of TSC include benign tumours of the brain (so-called cortical tubers or subependymal giant cell astrocytoma), skin or kidney. Approximately 90% of patients suffer from epilepsy, often drug-resistant, occurring very early in life. The treatment of TSC is largely symptomatic and symptom-specific. In the treatment of epilepsy, drugs and approaches commonly used to treat epilepsy in infants are used. The most used drug is vigabatrin, which is, however, insufficient to achieve significant improvement in approximately 50% of patients. The need to include additional drugs (usually one or two more) defines epilepsy as drug-resistant. However, if inhibition of epilepsy is achieved with treatment, after a minimum of 24 months, the attending physician can decide to withdraw the medication gradually. In the case of TSC, about 50% of patients experience a recurrence of epileptic seizures during or shortly after the drug withdrawal process, which is a major stress for both the patient and their caregivers and results in need to resume treatment. Similar problems exist with many childhood epilepsies. Currently, there are no molecular markers for predicting whether patients with drug-controlled epilepsy, including those with TSC, will experience a relapse of epileptic seizures if antiepileptic drugs are discontinued.

The invention aimed to provide molecular markers for the prediction of the epilepsy recurrence in patients after drug withdrawal.

The subject of the invention is a panel of RNA markers for the prediction of the epilepsy recurrence following drug withdrawal in patients with tuberous sclerosis, characterised in that it comprises following markers P2RY13, IL13RA1, PDE4B.

Preferably, the panel according to the invention comprises at least one RNA marker selected from PTGS2, RGCC, PPP1R3F, TLE2, LZTS3, SERINC3, TMEM238, and the tuberous sclerosis patient is male.

Another subject of the invention is an in vitro method to predict the epilepsy recurrence following drug withdrawal in patients with tuberous sclerosis, comprising the following steps:
a) Measurement of the expression level of a panel of predictive markers prior to drug withdrawal;
b) Determination of the risk of epilepsy recurrence based on the level of the of markers from the prediction panel,
characterised in that said panel of predictive markers is a panel of RNA markers comprising P2RY13, IL13RA1, PDE4B, and the determination of the risk of epilepsy recurrence after drug withdrawal is based on altered levels of at least two predictive markers of the said panel compared to a reference level.

Preferably, the measurement of the expression level and the determination of the risk of epileptic recurrence is carried out based on all three markers from the panel of predictive markers.

Preferably, in step a) the measurement is performed by qRT-PCR, ddPCR or targeted RNA sequencing by the next-generation sequencing (NGS) method.

Preferably, the test material is peripheral blood.

Preferably, the tuberous sclerosis patient is male.

Preferably, the predictive marker panel comprises at least one marker selected from PTGS2, RGCC, PPP1R3F, TLE2, LZTS3, SERINC3, TMEM238.

Another subject of the invention is using a panel of markers according to the invention, for predicting the epilepsy recurrence following drug withdrawal in patients with tuberous sclerosis according to the method of the invention.

The invention provides the following advantages:
- Prediction of epilepsy recurrence after drug withdrawal by the method according to the invention is carried out before drug withdrawal and will therefore minimise unnecessary exposure of patients to epilepsy recurrence after drug withdrawal;
- Allowance of safe withdrawal of drugs that are expensive and cause side effects (e.g., nephrotoxicity)
- Increasing the quality of life for patients and their caretakers.

### A detailed description of the invention:

Descriptions of embodiments of the invention in the present application are provided by way of example and are not intended to limit the scope of the invention. The described embodiments comprise different features, not all of which are required in all embodiments of the invention. Some embodiments utilize only some of the features or possible combinations of the features. Variations of embodiments of the invention that are described, and embodiments of the invention comprising different combinations of features noted in the described embodiments, will come to the mind of those who are experts in the field. The scope of the invention is limited only by the claims.

Moreover, within the meaning of this description, the phrase "in an embodiment" and "in certain embodiment" is to be understood as in one or more embodiments. Further, features present in the various embodiments may be combined with each other.

In the first aspect, the invention relates to a panel of RNA markers for the prediction of the epilepsy recurrence following drug withdrawal in patients with tuberous sclerosis that comprises the following markers P2RY13, IL13RA1, PDE4B. Wherein the panel according to the invention can be used regardless of the patient's gender.

In the meaning of invention, a predictive biomarker for the occurrence of epilepsy after drug withdrawal by patients with TSC is understood as change in RNA level of said biomarker before the occurrence of epilepsy at the clinical level while the patient is still taking regular doses of drugs, thus minimalizing unnecessary exposure of patients to epilepsy recurrence after drug discontinuation.

The efficacy of the RNA markers according to the invention has been demonstrated experimentally. Patients from TSC with drug-controlled epilepsy were recruited for the study (approximately 30 patients: girls and boys; aged 3-14 years). Patients had their blood drawn for RNA-based analyses at the time of the decision to discontinue the drug but before the drug was discontinued. Patients were then monitored for the epilepsy recurrence to assign them to a seizure-free (N) or epilepsy recurrence (R) group.

Unexpectedly, the inventors of the invention showed that TSC patients (regardless of gender) who developed epilepsy recurrence after drug withdrawal (i.e., the R group) had reduced RNA levels of a panel of markers according to the invention, i.e., P2RY13, IL13RA1, PDE4B, relative to those in the N group before drug withdrawal, confirming their applicability as predictive markers. To minimise the risk to the patient as much as possible, at least two of the three markers from the panel in any combination should be used for the prediction of epilepsy recurrence.

In one embodiment, the prediction of the epilepsy recurrence following drug withdrawal in patients with TSC is based on reduced expression levels of two predictive markers from the predictive panel compared to the reference level.

In one embodiment, the prediction of epilepsy recurrence following drug withdrawal in patients with TSC is based on reduced expression levels of the P2RY13 marker and IL13RA1. In another embodiment, the prediction of epilepsy recurrence following drug withdrawal in patients with TSC is based on reduced expression levels of the P2RY13 marker and PDE4B. In another embodiment, the prediction of the epilepsy recurrence following drug withdrawal in patients with TSC is based on reduced expression levels of the marker IL13RA1 and PDE4B.

In a preferred embodiment, the prediction of the occurrence of epilepsy recurrence following drug withdrawal in patients with TSC is based on reduced expression levels of all three predictive markers from the predictive panel compared to the reference level.

In one embodiment, the reference level comprises patients with TSC without epilepsy recurrence following drug withdrawal. Advantageously, these are patients of similar or the same age as those in the tested group.

In another embodiment, the prediction analysis of the epilepsy recurrence following drug withdrawal in patients with TSC is based on a comparison of the median expression of predictive markers from the predictive panel according to the invention compared to the median of the reference level.

In another embodiment, for male patients with TSC, the predictive panel according to the invention is expanded to include at least one RNA marker selected from PTGS2, RGCC, PPP1R3F, TLE2, LZTS3, SERINC3, TMEM238. In one embodiment, the prediction panel according to the invention is extended with one RNA marker selected from PTGS2, RGCC, PPP1R3F, TLE2, LZTS3, SERINC3, TMEM238. However, according to the invention, any number of markers may also be selected, e.g., two, three, five or all markers from a male-specific additional panel.

Unexpectedly, the inventors of the invention showed that for male TSC patients with epilepsy recurrence, reduced levels of PTGS2, SERINC3 as well as increased levels of RGCC, PPP1R3F, TLE2, LZTS3, TMEM238, were observed before drug withdrawal. Similarly, the efficacy of these markers has been demonstrated experimentally. Patients with TSC with drug-controlled epilepsy were recruited for the study (approximately 30 patients-girls and boys; aged 3-14 years). Patients had their blood drawn for RNA-based analyses at the time of the decision to discontinue the drug but before the drug was discontinued. Patients were then monitored for the epilepsy recurrence to assign them to a seizure-free (N) or epilepsy recurrence (R) group. The specificity of the PTGS2, RGCC, PPP1R3F, TLE2, LZTS3, SERINC3, TMEM238 panel was demonstrated as a panel of predictive markers of epilepsy recurrence after drug withdrawal in male subjects with TSC. Wherein for this extended panel, male patients with TSC without epilepsy recurrence were used as a reference.

In a preferable embodiment, the test material is peripheral blood taken from patients with TSC while they are still taking antiepileptic drugs.

In some embodiments, the panel markers according to the invention are mRNA markers.

In some embodiments, measurement of the level of one or more predictive markers from the panel of markers according to the invention is performed in vitro using qRT-PCR, ddPCR or targeted RNA sequencing by NGS.

In some embodiments, the measurement of the level of one or more predictive markers from the panel of markers according to the invention is detected in vitro by, for example, immunoassay (ELISA), radioimmunoassay, radioimmunoassay, western blot, Southern blot, Northern blot, in situ hybridization, immunoprecipitation, mass spectrometry, RT-PCR, PCR, enzyme activity assay, combinations thereof, or other techniques known in the field.

In the second aspect, the invention relates to an in vitro method to predict the epilepsy recurrence following drug withdrawal in patients with tuberous sclerosis, comprising the following steps:
a) Measurement of the expression level of a panel of predictive markers prior to drug withdrawal;
b) Determination of the risk of epilepsy recurrence based on the level of the markers from the prediction panel,
wherein the said panel of predictive markers is a panel of RNA markers comprising P2RY13, IL13RA1, PDE4B, and the determination of the risk of epilepsy recurrence after drug withdrawal is based on altered levels of at least two predictive markers of the said panel compared to a reference level.

In a preferable embodiment, the test material is peripheral blood taken from patients with TSC while they are still taking antiepileptic drugs.

In some embodiments, the panel markers used in the method according to the invention are mRNA markers.

In some embodiments, the measurement of the level of one or more predictive markers from the panel of markers according to the invention is performed in vitro using qRT-PCR, ddPCR or targeted RNA sequencing by NGS.

In some embodiments, the measurement of the level of one or more predictive markers from the panel of markers according to the invention is detected in vitro by, for example, immunoassay (ELISA), radioimmunoassay, radioimmunoassay, western blot, Southern blot, Northern blot, in situ hybridization, immunoprecipitation, mass spectrometry, RT-PCR, PCR, enzyme activity assay, combinations thereof, or other techniques known in the field.

Wherein, said altered level of markers from the panel according to the invention represents a reduced level (e.g., expression level, amount of RNA) of markers from the panel relative to a reference level.

In one embodiment, the prediction of the epilepsy recurrence following drug withdrawal in patients with TSC is based on the reduced expression level of two predictive markers from the predictive panel compared to a reference level.

In one embodiment, the prediction of epilepsy recurrence following drug withdrawal in patients with TSC is based on reduced expression levels of the marker P2RY13 and IL13RA1.

In a preferable embodiment, the prediction of the epilepsy recurrence following drug withdrawal in patients with TSC is based on reduced expression levels of all three predictive markers from the predictive panel compared to the reference level.

In one embodiment, the reference level comprises the expression level in patients with TSC without epilepsy recurrence. Advantageously, these are patients of similar or the same age.

In another embodiment, the prediction analysis of the epilepsy recurrence following drug withdrawal in patients with TSC is based on a comparison of the median expression of predictive markers from the predictive panel compared to the median of the reference level.

In another embodiment for male TSC patients, the predictive panel according to the invention is expanded to include at least one RNA marker selected from PTGS2, RGCC, PPP1R3F, TLE2, LZTS3, SERINC3, TMEM238. In one embodiment, the prediction panel according to the invention is extended with one RNA marker selected from PTGS2, RGCC, PPP1R3F, TLE2, LZTS3, SERINC3, TMEM238. However, in the meaning of the invention, any number of markers may also be selected, e.g., two, three, five or all markers from a male-specific additional panel. Whereby, for these embodiments, expression levels of these markers in male TSC patients without epilepsy recurrence after drug withdrawal are used as reference levels. These are preferably patients of similar or the same age.

In another aspect, the invention relates to the use of a panel of markers according to the invention, for predicting the epilepsy recurrence following drug withdrawal in patients with tuberous sclerosis according to the method according to the invention.

The invention is presented in exemplary embodiments and illustrated in the drawing in which: Fig. 1 is a volcano plot showing RNA differentially expressed in the N and R groups of TSC patients without gender split; Fig. 2 is a volcano plot showing RNA differentially expressed in groups N and R in boys with TSC; Fig. 3 shows a comparison of RNA expression of the P2RY13 marker in the group of TSC patients with epilepsy recurrence (R) and in the group without epilepsy recurrence (N); Fig. 4 shows a comparison of RNA expression of the marker IL13RA1 in the group of TSC patients with epilepsy recurrence (R) and in the group without epilepsy recurrence (N); Fig. 5 shows a comparison of RNA expression of the PDE4B marker in the group of TSC patients with epilepsy recurrence (R) and in the group without epilepsy recurrence (N); Fig. 6 shows a comparison of RNA expression of the PTGS2 marker in the group of male TSC patients in the group of TSC patients with epilepsy recurrence (R) and in the group without epilepsy recurrence (N); Fig. 7 shows a comparison of RNA expression of the RGCC marker in the group of male TSC patients with epilepsy recurrence (R) and in the group without epilepsy recurrence (N); Fig. 8 shows a comparison of RNA expression of the marker PPP1R3F in the group of male TSC patients with epilepsy recurrence (R) and in the group without epilepsy recurrence (N); Fig. 9 shows a comparison of RNA expression of the TLE2 marker in the group of male TSC patients with epilepsy recurrence (R) and in the group without epilepsy recurrence (N); Fig. 10 shows a comparison of RNA expression of the marker LZTS3 in the group of male TSC patients with epilepsy recurrence (R) and in the group without epilepsy recurrence (N); Fig. 11 shows a comparison of RNA expression of the marker SERINC3 in the group of male TSC patients with epilepsy recurrence (R) and in the group without epilepsy recurrence (N); Fig. 12 shows a comparison of RNA expression of the marker TMEM238 in the group of male TSC patients with epilepsy recurrence (R) and in the group without epilepsy recurrence (N).

The following exemplary embodiments are for illustrative purposes and are not intended to limit the present invention in any way.

### Example 1.

### Confirmation of the properties of a marker panel according to the invention

The present example provides a methodology used to validate the selection of an RNA from the panel according to the invention comprising the following markers: P2RY13, IL13RA1, PDE4B, as a panel of predictive markers for predicting the occurrence of epilepsy recurrence following drug withdrawal in patients with tuberous sclerosis.

Further, this example provides the methodology used to validate the selection of an RNA from a panel according to the invention comprising the following markers: PTGS2, RGCC, PPP1R3F, TLE2, LZTS3, SERINC3, TMEM238, as a predictive marker panel for predicting the occurrence of epilepsy recurrence following drug withdrawal in male patients with tuberous sclerosis.

### Materials and methods

### Patients.

TSC patients with drug-controlled epilepsy were recruited for the study (30 patients: aged 3-14 years). Patients had their blood drawn for analysis at the time of the decision to discontinue the treatment but before the drug was discontinued. Patients were then monitored for epilepsy recurrence for 12 months to assign to a seizure-free group (N; 20 patients, including 11 boys and 9 girls) or an epilepsy recurrence group (R; 10 patients, including 6 boys and 4 girls).

### RNA Isolation

The starting material for RNA isolation was whole peripheral blood. Blood was not taken while fasting or at a specific time. 2.5 ml was collected directly into Qiagen Paxgene RNA Tubes (Qiagen; cat. no. 762165). RNA isolation was performed using the PAXgene Blood RNA Kit (50) (Qiagene; Cat. No.: 762164). The quality of RNA for RNAseq was checked by determining the RNA integrity number (RIN). This parameter was determined using an Agilent 2100 Bioanalyzer and dedicated Agilent RNA 6000 Nano Kit reagent kits. Libraries were prepared from those samples whose RIN was greater than 7 (on a 0-10 scale).

### Preparation of libraries for mRNA sequencing with rRNA depletion

The procedure for preparing libraries for RNA sequencing after ribosomal depletion was performed using the NEBNext Ultra II Directional RNA Library Prep Kit and NEBNext rRNA Depletion Kit (Human/Mouse/Rat) (NEB Ltd) according to the manufacturer's instructions. The method for rRNA depletion from the total RNA pool is based on RNAase H, which discards mitochondrial and cytoplasmic rRNA. 500 ng of total RNA was used as starting material in the procedure. The next step after rRNA depletion is thermal fragmentation of the material. In the next step, RNA is transcribed into cDNA, the first and second strand of cDNA is synthesized sequentially. The first strand of cDNA is obtained using primers that attach randomly to all regions of the RNA (random primers). In this way, a transient cDNA-RNA hybrid structure is obtained, which is then transformed into a double-stranded cDNA with simultaneous incorporation of dUTP into the second strand of cDNA. Strand specificity is obtained during complementary strand synthesis.

The resulting double-stranded cDNA was purified using AMPure XP magnetic beads. After removing the alcohol and drying the beads at room temperature, the beads were suspended in the reaction mixture for ligation. This step involves the ligation of adaptor sequences, which due to their loop structure must then be cut by the dedicated USER enzyme (NEB, Ipswich, MA, USA) to allow the final amplification of the library to occur. To obtain sufficient library concentrations, 10 cycles of PCR reactions were performed (the number of PCR cycles depends on the input RNA amount) according to the protocol in the table. Importantly, the second synthesized DNA strand containing dUTP is not amplified because the polymerase is specific only to the nucleotides present in the DNA. Consequently, this ensures that reads derived directly from transcripts are always strand-matched with R2 reads derived from paired-end sequencing.

**Table 1. PCR reaction parameters used for the final amplification of the library obtained using the NEBNext rRNA Depletion Kit.**

| **Step name** | **Temperature** | **Duration** |
|---|---|---|
| **Step I** | 98°C | 30 sec |
| **Step II (6-16 cycles)** | 98°C | 15 sec |
| | 65°C | 75 sec |
| **Step III** | 65°C | 5 min |
| **Step IV** | 4°C | ∞ |

After PCR, AMPure XP beads were added to the reaction mixture in a 1:1 ratio as described above. The library DNA was then recovered from the AMPure XP beads by eluting with 10 mM Tris-HCl solution.

The final concentration of the resulting library and its purity expressed as A260/280 values (range of normal parameter values 1.8-2.2) were measured using a Nanodrop instrument (Thermo Scientific) as well as using a Quantus Fluorometer instrument and its dedicated QuantiFluor dsDNA System reagent kit (Promega, Madison, Wisconsin, USA). Library quality assessment was performed using an Agilent 2100 Bioanalyzer and a dedicated Agilent High Sensitivity DNA Kit reagent kit. Libraries were sequenced on a HiSeq1500 (Illumina, San Diego, USA) in Rapid Run paired-end mode (2x76 cycles).

### RNAseq secondary/tertiary analysis

Trimmomatic (version 0.36), with default options, was used to remove Illumina-specific adapters, low-quality 5' and 3' bases, and short reads from FASTQ files. The RNA sequencing reads were then mapped to a reference human genome sequence (hg38) using the STAR algorithm (version 2.6.1b), with the twopassMode Basic option enabled to increase read mapping to novel junctions identification. Picard Tools (version 2.17.1) was then used to identify and mark duplicate reads. Using featureCounts software (version 1.5.3), the resulting RNAseq mapped reads were summed and counted by genes in paired (-p), reversely stranded mode (-s 2), and only reads with MAPQ values of 3 or higher were considered. For downstream analysis, samples with fewer than 10 million paired reads were deleted.

Next, low-expressed genes were pre-filtered (raw counts of at least 20 in all analyzed datasets), and the resulting RNA-seq dataset was normalized using the conditional quantile normalization method (version 1.32.0), followed by DESeq2 differentially expressed genes analysis (version 1.24.0). We used the DESeqDataSetFromMatrix function to adjust for gender effect in our first attempt to find differentially expressed genes; nevertheless, we found that the outcome was still heavily influenced by gender and not by the treatment response. As a result, we divided our data by gender and ran two separate differential expression analyses for males and females.

Standard differential expression analyses were performed using the DESeq function, with the "without seizures (N)" group as a reference and compared with the "with recurrent seizures (R)" group. The variance stabilizing transformation (vst) function in DESeq2 was used to transform the data for visualization. Only statistically significant genes were selected (q-value ≤ 0.05), and multiple testing was controlled by the Bonferroni-Hochberg (BH) method. In addition to the degree of significance, potential biomarkers were selected based on two factors: (1) the distribution of normalized abundances, in which we looked for low within-group variability in expression, and (2) fold change between compared groups, in which greater log2 FC was prioritized. We also performed two independent differential expression analyses in men and women, using the "seizure-free (N)" group as the reference group and comparing it with the "recurrent seizure (R)" group. In this case, only statistically significant genes were selected (q-value ≤ 0.05) and the BH method was used to control for multiple testing.

### Results

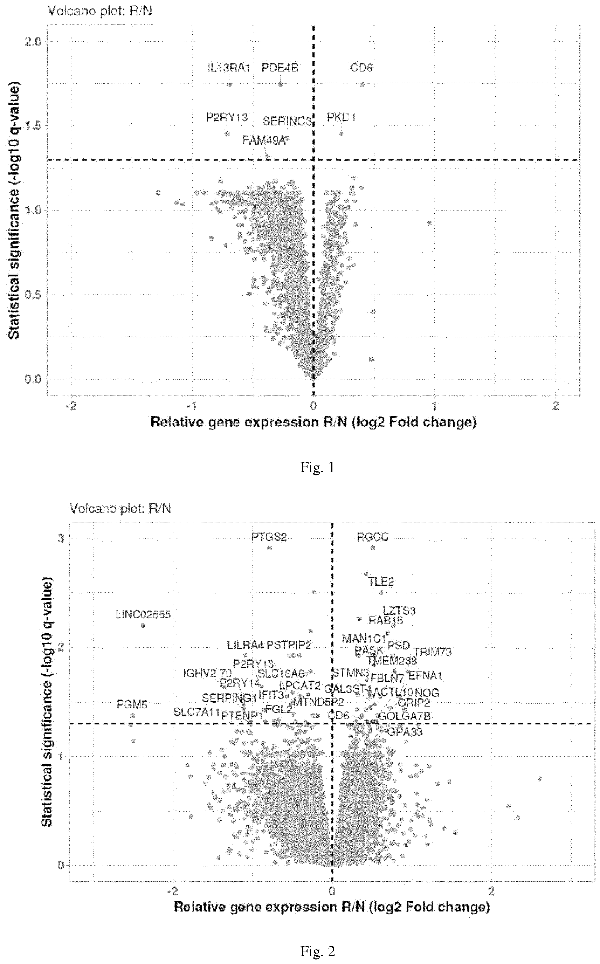

As indicated in Fig. 1, data analysis performed without gender split revealed 7 RNAs differentially expressed between N and R groups.

When the data were separated by gender, no genes were differentially expressed in girls between the groups. However, in the case of boys, 66 genes were differentially expressed between the tested groups (Fig. 2).

As a result of the analysis, considering the criteria described above, we selected RNA sets that could be predictive biomarkers of epilepsy recurrence before drug withdrawal. Two groups of biomarkers, one predictive for both sexes (P2RY13, IL13RA1, PDE4B) and one specific only for boys (PTGS2, RGCC, PPP1R3F, TLE2, LZTS3, SERINC3, TMEM238) were selected. Detailed results in the form of normalized mapped fragment counts for a given gene, along with statistical significance (Adj.p) are discussed below:

### Potential predictive biomarkers of epilepsy recurrence in patients with TSC without gender split:

### P2RY13 marker

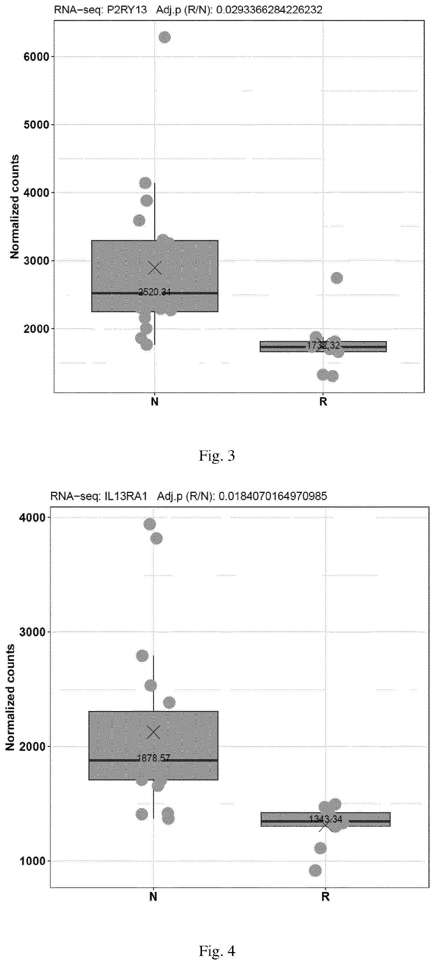

As indicated in Fig. 3, the level of P2RY13 mRNA in the blood of TSC patients collected before discontinuation of antiepileptic treatment is lower in those subjects who had a epilepsy recurrence after drug withdrawal (R) compared to patients without epilepsy recurrence. Low blood P2RY13 mRNA levels may correlate with the likelihood of epilepsy recurrence in TSC patients after treatment withdrawal and thus potentially be an indication for treatment continuation.

### Marker IL13RA1

As indicated in Fig. 4, the mRNA level of IL13RA1 in the blood of TSC patients collected before discontinuation of antiepileptic treatment is lower in those subjects who had a epilepsy recurrence after drug withdrawal (R) compared with patients without epilepsy recurrence. Low blood IL13RA1 mRNA levels may correlate with the likelihood of epilepsy recurrence in TSC patients after treatment withdrawal and thus potentially be an indication for treatment continuation.

### PDE4B marker

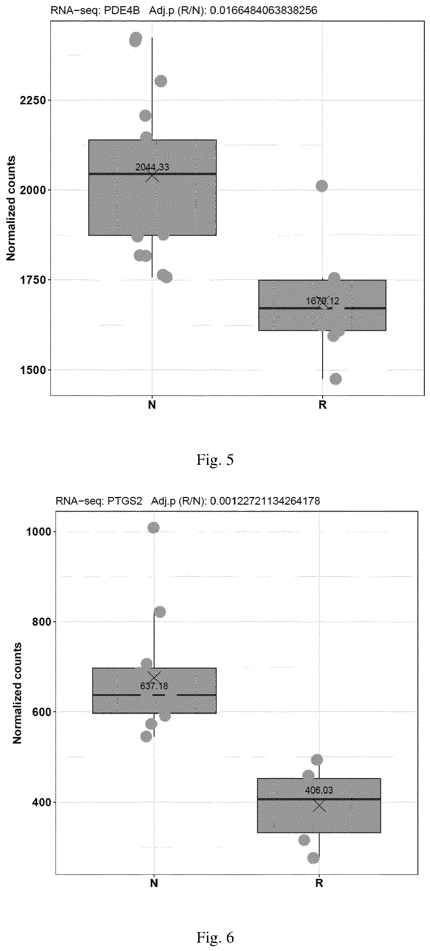

As indicated in Fig. 5, the level of PDE4B mRNA in the blood of TSC patients collected before a withdrawal of antiepileptic treatment is lower in those patients who had a epilepsy recurrence after drug withdrawal (R) than in patients without epilepsy recurrence. Low blood levels of PDE4B mRNA may correlate with the likelihood of epilepsy recurrence in patients with TSC after treatment withdrawal and thus potentially be an indication for treatment continuation.

### Potential biomarkers of epilepsy recurrence in a boys-only group of TSC patients:

### PTGS2 marker

As indicated in Fig. 6, the mRNA level of PTGS2 in the blood of boys with TSC taken before a withdrawal of antiepileptic drugs is lower in those who had a epilepsy recurrence after drug withdrawal (R) than in patients without epilepsy recurrence. Low blood levels of PTGS2 mRNA may correlate with the likelihood of epilepsy recurrence in patients with TSC after treatment withdrawal and thus potentially be an indication for treatment continuation.

### RGCC marker

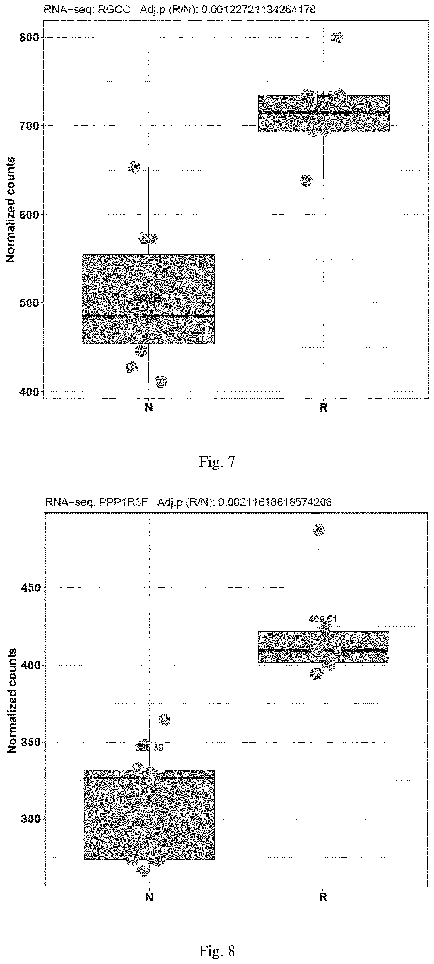

As indicated in Fig. 7, RGCC mRNA levels in blood from boys with TSC collected before antiepileptic drug withdrawal are higher in those who had a epilepsy recurrence (R) after drug withdrawal than in patients without the epilepsy recurrence. High levels of RGCC mRNA in the blood may correlate with the likelihood of epilepsy recurrence in patients with TSC after treatment withdrawal and thus potentially be an indication for treatment continuation.

### PPP1R3F marker

As indicated in Fig. 8, the mRNA level of PPP1R3F in the blood of boys with TSC collected before a withdrawal of antiepileptic drugs is higher in those who had a epilepsy recurrence (R) after drug withdrawal than in patients without epilepsy recurrence. High PPP1R3F mRNA levels in the blood may correlate with the likelihood of epilepsy recurrence in TSC patients after treatment withdrawal and thus potentially be an indication for treatment continuation.

### TLE2 marker

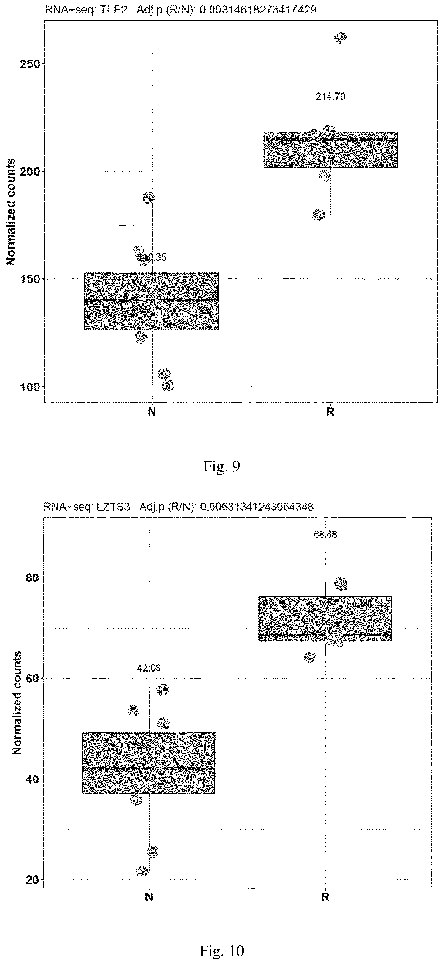

As indicated in Fig. 9, TLE2 mRNA levels in the blood of boys with TSC collected before a withdrawal of antiepileptic drugs are higher in those who had a epilepsy recurrence (R) after drug withdrawal than in patients without epilepsy recurrence. High blood TLE2 mRNA levels may correlate with the likelihood of epilepsy recurrence in patients with TSC after treatment withdrawal and thus potentially be an indication for treatment continuation.

### Marker LZTS3

As indicated in Fig. 10, the mRNA level of LZTS3 in the blood of boys with TSC collected before a withdrawal of antiepileptic drugs is higher in those who had a epilepsy recurrence (R) after drug withdrawal compared with patients without epilepsy recurrence. High blood LZTS3 mRNA levels may correlate with the likelihood of epilepsy recurrence in patients with TSC after treatment withdrawal and thus potentially be an indication for treatment continuation.

### SERINC3 marker

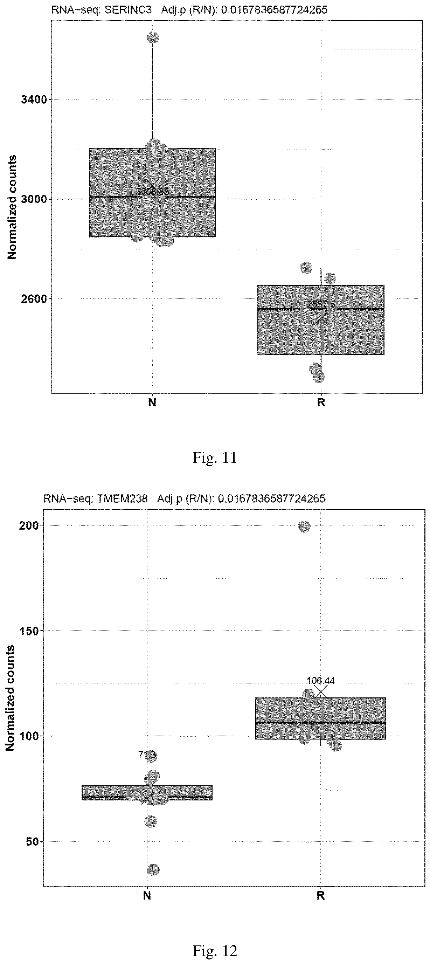

As indicated in Fig. 11, SERINC3 mRNA levels in blood from boys with TSC collected before a withdrawal of antiepileptic drugs are lower in those who had a epilepsy recurrence (R) after drug withdrawal than in patients without epilepsy recurrence. Low SERINC3 mRNA levels in the blood may correlate with the likelihood of epilepsy recurrence in patients with TSC after treatment withdrawal and thus potentially be an indication for treatment continuation.

### Marker TMEM238

As indicated in Fig. 12, TMEM238 mRNA levels in the blood of boys with TSC collected before a withdrawal of antiepileptic drugs are higher in those who had a epilepsy recurrence (R) after drug withdrawal compared with patients without epilepsy recurrence. High blood TMEM238 mRNA levels may correlate with the likelihood of epilepsy recurrence in patients with TSC after treatment withdrawal and thus potentially be an indication for treatment continuation.

The conducted analysis demonstrated the usefulness of all markers from the panel according to the invention in predicting the occurrence of epilepsy recurrence in TSC patients after drug withdrawal. Therefore, a general-use panel and a panel specific to male patients were selected. Prediction of epilepsy recurrence after drug withdrawal using markers according to the invention by the method according to the invention is carried out before drug withdrawal. Therefore, it will minimize unnecessary exposure of patients to epilepsy recurrence after drug withdrawal and will allow safe discontinuation of drugs that are both expensive and have side effects. The use of the panel according to the invention will therefore have wide application in medicine.

### Example 2.

In this example, the marker panel according to the invention used to determine the risk of epilepsy recurrence in a female patient with TSC after antiepileptic drugs withdrawal is based on the following two RNA markers, P2RY13 and IL13RA1. The collection of a peripheral blood sample for the study and the entire prediction analysis is performed when the female patient continues to take antiepileptic drugs.

Sample collection from the patient, RNA isolation and sequencing were performed as in Example 1. In this example of implementation, the measurement of the expression levels of the P2RY13 and IL13RA1 markers from the panel according to the invention was performed by RNA sequencing by NGS, while according to the invention, the determination of the RNA marker expression can also be performed by another technique known in the field, for example by qRT-PCR or ddPCR.

Next, the risk of epilepsy recurrence in the patient was determined based on the level of the predictive markers P2RY13 and IL13RA1 against a reference level. The reference level was the expression level of the markers in the blood of a female or male patient of similar age with TSC who has not had a recurrence of epilepsy in the future after withdrawal of drugs. In this example of implementation, the classification of the tested patient into R or N groups relaying on P2RY13 and IL13RA1 mRNA quantity is based on comparing the median expression levels of each of these predictive markers to the median reference level for the given marker. Drug discontinuation is not recommended if a patient is classified in the R group.

### Example 3.

In this example, the marker panel according to invention used to determine the risk of epilepsy recurrence in a female patient with TSC, after discontinuation of antiepileptic drugs is based on all RNA markers P2RY13, IL13RA1, PDE4B. The collection of a peripheral blood sample for the study and the entire predictive analysis is performed when the patient continues to take antiepileptic drugs.

Sample collection from the patient, RNA isolation and sequencing were performed as in Example 1. In this implementation example, the measurement of the P2RY13, IL13RA1 and PDE4B RNA marker (according to the invention) expression was done by RNA NGS. According to the invention, the RNA marker expression measurement can also be performed by another technique known in the field, for example, qRT-PCR or ddPCR.

The risk of epilepsy recurrence in the female patient was then determined based on the marker levels of all three predictive markers against a reference level. The reference level was the expression level of the markers in the blood of a female or male patient of similar age with TSC who has not had a recurrence of epilepsy in the future after withdrawal of drugs. In this example of implementation, the classification of the tested patient into R or N groups based on the expression of the panel of three predictive RNA markers is based on a comparison of the median expression of each of these predictive markers to the median reference level for the given marker. Drug discontinuation is not recommended if a patient is classified in the R group.

### Example 4.

In this example, a marker panel according to the invention is used to determine the risk of epilepsy recurrence in a male patient with TSC after drug withdrawal. The panel is based on the following two general panel RNA markers: P2RY13, IL13RA1 and one male-specific marker: PTGS2. The collection of a peripheral blood sample for testing and all predictive analysis are performed while the patient continues to take antiepileptic drugs.

In this implementation example, a single RNA marker from a male-specific panel is used, whereas it is understood by the invention that for the prediction of epilepsy recurrence in male patients with TSC, any number of markers from the group consisting of PTGS2, RGCC, PPP1R3F, TLE2, LZTS3, SERINC3, TMEM238 may be used, with at least two markers selected from the group consisting of: P2RY13, IL13RA1, PDE4B.

Sample collection from the patient, RNA isolation and sequencing were performed as in Example 1. In this example of implementation, the measurement of the RNA markers expression was done by RNA NGS. According to the invention, the measurement of the RNA markers' expression can also be performed by another technique known in the field, e.g., qRT-PCRT-PCR or ddPCR.

Next, the risk of epilepsy recurrence in the patient was determined based on the marker levels of all three predictive markers against a reference level. The reference level was the expression level of the markers in the blood of a male patient of similar age with TSC who has not had a recurrence of epilepsy in the future after withdrawal of drugs. In this example implementation, the classification of the test patient into R or N groups based on the mRNA quantity of a panel of three predictive markers is based on comparing the median expression of each of these predictive markers to the median of the reference level. Drug discontinuation is not recommended if a patient is classified in the R group.

## Claims

1. A panel of RNA markers for the prediction of the epilepsy recurrence following drug withdrawal in patients with tuberous sclerosis, **characterised in that** it comprises following markers P2RY13, IL13RA1, PDE4B.

2. The panel according to claim 1, **characterised in that** it comprises at least one RNA marker selected from PTGS2, RGCC, PPP1R3F, TLE2, LZTS3, SERINC3, TMEM238, and the tuberous sclerosis patient is male.

3. An in vitro method to predict the epilepsy recurrence following drug withdrawal in patients with tuberous sclerosis, comprising the steps of:
a) Measurement of the expression level of a panel of predictive markers prior to drug withdrawal;
b) Determination of the risk of epilepsy recurrence based on the level of the of markers from prediction panel,
**characterised in that** said panel of predictive markers is a panel of RNA markers comprising P2RY13, IL13RA1, PDE4B, and the determination of the risk of epilepsy recurrence after drug withdrawal is based on altered levels of at least two predictive markers of said panel compared to a reference level.

4. The method according to claim 3, **characterised in that** the measurement of the expression level and the determination of the risk of epileptic recurrence is carried out based on all three markers from the panel of predictive markers.

5. The method according to any of the preceding claims 3 to 4, **characterised in that** in step a) the measurement is performed by qRT-PCR, ddPCR or targeted RNA sequencing by NGS method.

6. The method according to any of the preceding claims 3 to 5, **characterised in that** the test material is peripheral blood.

7. The method according to any of the preceding claims 3 to 6, **characterised in that** the tuberous sclerosis patient is male.

8. The method according to claim 7, **characterised in that** the predictive marker panel comprises at least one marker selected from PTGS2, RGCC, PPP1R3F, TLE2, LZTS3, SERINC3, TMEM238.

9. Use of a panel of markers according to claim 1 or 2, for predicting the epilepsy recurrence following drug withdrawal in patients with tuberous sclerosis according to the method of any of the preceding claims 3 to 8.
